# EUROPEAN PATENT SPECIFICATION

(11) **EP 1 489 169 B1**
(45) Date of publication and mention of the grant of the patent: **08.07.2009**
(21) Application number: 03707041.4
(22) Date of filing: 25.02.2003
(51) Int. Cl.: C12N 15/09, C12Q 1/02, C12Q 1/66, A61K 45/00, A61P 35/00, G01N 33/574

(54) **SUMOYLATION INHIBITORS**
SUMOYLATION-INHIBITOREN
INHIBITEURS DE SUMOYLATION

(30) Priority: 01.03.2002 JP 2002055796; 18.09.2002 JP 2002271527
(43) Date of publication of application: 22.12.2004
(73) Proprietor: Daiichi Sankyo Company, Limited, Chuo-ku Tokyo (JP)
(72) Inventor: KIKUCHI, Akira, Hiroshima 732-0814 (JP); YAMAMOTO, Hideki, Hiroshima 733-0032 (JP); WAKITA, Kenichi, DAIICHI PHARMACEUTICAL CO., LTD., Tokyo 134-8630 (JP)
(74) Representative: Krauss, Jan
(86) International application number: PCT/JP2003/002029
(87) International publication number: WO 2003/074695

(56) References cited:
- KADOYA TAKAYUKI ET AL: "Desumoylation activity of Axam, a novel axin-binding protein, is involved in downregulation of beta-catenin" MOLECULAR AND CELLULAR BIOLOGY, vol. 22, no. 11, June 2002 (2002-06), pages 3803-3819, XP002369642 ISSN: 0270-7306
- YAMAMOTO HIDEKI ET AL: "Sumoylation is involved in beta-catenin-dependent activation of Tcf-4." EMBO (EUROPEAN MOLECULAR BIOLOGY ORGANIZATION) JOURNAL, vol. 22, no. 9, 1 May 2003 (2003-05-01), pages 2047-2059, XP002369643 ISSN: 0261-4189
- DATABASE GENBANK NCBI; 31 October 2000 (2000-10-31), XP002369650 Database accession no. NM_003199
- DATABASE GENBANK NCBI; 25 April 2001 (2001-04-25), XP002369651 Database accession no. NM_016166
- SACHDEV S. ET AL.: 'PIASy, a nuclear matrix-.associated SUMO E3 ligase, represses LEF1 activity by sequestration into nuclear bodies' GENES DEV. vol. 15, no. 23, 2001, pages 3088 - 3103, XP002966327
- KORINEK V. ET AL.: 'Constitutive transcriptional activation by a beta-catenin-Tcf complex in APC-/-colon carcinoma' SCIENCE vol. 275, no. 5307, 1997, pages 1784 - 1787, XP002070159
- MORIN J. ET AL.: 'Activation of beta-catenin-Tcf signaling in colon cancer by mutations in beta-catenin or APC' SCIENCE vol. 275, 1997, pages 1787 - 1790, XP002939377
- KINZLER K.W. ET AL.: 'Lessons from hereditary colorectal cancer' CELL vol. 87, no. 2, 1996, pages 159 - 170, XP002153982
- AKIRA KIKUCHI ET AL.: 'SUMO-ka o kaisuru Wnt signal dentatsu kiko no seigyo' SEIKAGAKU vol. 75, no. 8, 25 August 2002, page 640, XP002968429

## Description

### TECHNICAL FIELD

The present invention relates to a compound or inhibitor that inhibits both the sumoylation of Tcf-4 by a protein of the protein inhibitor of activated STAT (PIAS) family and the enhanced transcriptional activity of Tcf-4 induced thereby, a desumoylation agent for Tcf-4, and a method for identifying the compound. The present invention also relates to a preventive and/or remedy for colon cancer and a method for preventing and/or treating colon cancer that comprises inhibiting the sumoylation of Tcf-4 induced by a PIAS family protein and/or desumoylating a sumoylated Tcf-4.

### BACKGROUND ART

The term "SUMO" is an abbreviation of "small ubiquitin-like modifier", and refers to a protein that can be used like a ubiquitin for protein modification. The conjugation of SUMO with a target protein is termed "sumoylation". As target proteins of sumoylation, RanGAP1, PML, IκBα, p53 and the like are known.

Sumoylation is a phenomenon that has recently begun to draw attention, and the significance of sumoylation has not yet been elucidated (non-patent document 1). Some enzymes are known to be involved in the process of sumoylation. Such enzymes are different from those involved in the ubiquitination process (non-patent document 2).

There are at least three types of mammalian SUMOs, SUMO-1, SUMO-2, and SUMO-3. SUMO-1 is activated by an E1 enzyme (Aos/Uba2), then transferred to an E2 conjugation enzyme (Ubc9), and finally conjugated to a target protein by an E3 ligase (protein inhibitor of activated STAT (herein after, abbreviated as "PIAS") (non-patent documents 3 and 4) or RanBP2 (non-patent document 5)). Sumoylation is reversible, and desumoylation takes place when, e.g., a SUMO-specific protease is employed (non-patent document 6). Several SUMO-specific proteases, such as SENP family proteins, are known in mammals.

PIAS, which is the E3 ligase involved in sumoylation, is known to have family proteins. In mammals, four PIAS family proteins have been identified, PIASy, PIAS1, PIAS3, and PIASxα.

PIAS family proteins are involved in the intracellular signal transduction of cytokines and the like. For example, PIASy is a transcription factor of the Tcf family and sumoylates Lef-1 (Lymphoid enhancer factor 1) which works in the Wnt signaling pathway (non-patent document 4). Although PIASy suppresses the transcriptional activity of Lef-1, it does not affect its binding activity to DNA or β-catenin. On the other hand, Axam, which is identified as an Axin-associated protein, regulates the Wnt signaling pathway negatively by inducing the degradation of β-catenin (non-patent document 7). Axam is one of the SENP family proteins, and the regulatory functions of Axam in the Wnt signaling pathway require the desumoylation activity thereof (non-patent document 8).

PIAS1 and PIAS3 have been identified respectively as auxiliary factors that inhibit transcription control factors, STAT1 and STAT3, from binding to DNA (non-patent document 9). PIAS1 sumoylates the p53 tumor-suppressor gene product (non-patent document 3). PIASxα regulates androgen receptor (AR)-dependent transcription negatively by binding to AR (non-patent document 10).

As described above, it has been suggested that a target protein is sumoylated by the PIAS family proteins, which results in the functional alteration of the target protein. This has led to the PIAS family proteins receiving attention as sumoylation enzymes.

References cited herein are described below.
Non-patent document 1: Saitoh, T., Experimental Medicine (2001), vol. 19, p.112-119.
Non-patent document 2: Muller, S. et al., Nature Reviews Molecular Cell Biology (2001) 2: 202-210.
Non-patent document 3: Kahyo, T. et al., Molecular Cell (2001) 8: 713-718.
Non-patent document 4: Sachdev, S. et al., Genes and Development (2001) 15: 3088-3103.
Non-patent document 5: Kirsh, O. et al., EMBO Journal (2002) 21: 2682-2691.
Non-patent document 6: Yeh, E. et al., Gene (2000) 248: 1-14.
Non-patent document 7: Kadoya, T. et al., Molecular and Cellular Biology (2002) 22: 3803-3819.
Non-patent document 8: Kadoya, T. et al., Journal of Biological Chemistry (2000) 275: 37030-37037.
Non-patent document 9: Liu, B. et al., Proceedings of The National Academy of Sciences of The United States of America (1998) 95: 10626-10631.
Non-patent document 10: Kotaja, N. et al., Molecular Endocrinology (2000) 14: 1986-2000.
Non-patent document 11: Korinek, B., Science (1997) 275: 1784-1787.
Non-patent document 12: Morin, P.J. et al., Science (1997) 275: 1787-1790.
Non-patent document 13: Vogelstein, K. et al., Cell (1996) 87: 159-170.
Non-patent document 14: Tetsu, O. et al., Nature (1999) 398: 422-426.

### DISCLOSURE OF THE INVENTION

In the present invention, concentrated studies have been undertaken with the following aims: isolating a protein that is sumoylated by a protein of the PIAS family, regulating the sumoylation thereof, and providing means for controlling diseases resulting from the functional alteration of the protein caused by sumoylation. The present invention has been achieved by finding out that Tcf-4, a transcription factor, is sumoylated by PIASy, and that the Tcf-4 function is enhanced by sumoylation.

Accordingly, one aspect of the present invention relates to a method according to claim1 for identifying a compound that inhibits the sumoylation of Tcf-4 by PIAS, comprising the steps of: (i) contacting PIAS and/or Tcf-4 with a test compound under conditions allowing for the interaction of the test compound with PIAS and/or Tcf-4, and (ii) determining whether the test compound inhibits the sumoylation of Tcf-4 by PIAS, by employing a system that uses a signal and/or marker capable of detecting sumoylation of Tcf-4 to detect the presence, absence, or alteration of the signal and/or marker.

A further aspect of the present invention relates to a method according to claim 2 for identifying a compound that inhibits the sumoylation of Tcf-4 by PIAS, comprising the steps of: (i) using the cells described in (A), (B), (C), or (D), below, and contacting the cells with a test compound, and (ii) determining whether the test compound inhibits the sumoylation of Tcf-4 by PIAS, by detecting the sumoylated Tcf-4 or by detecting the presence, absence, or alteration of a signal and/or marker for detecting sumoylation of Tcf-4, wherein cells (A), (B), (C), and (D) are as follows:
(A): cells that have co-expressed PIAS, SUMO, and Tcf-4 (to which a signal and/or marker for detecting sumoylation of Tcf-4 is introduced),
(B): cells that have co-expressed PIAS, Tcf-4, and SUMO (to which a signal and/or marker for detecting sumoylation of Tcf-4 is introduced),
(C): cells that have co-expressed PIAS, Tcf-4 (to which a signal and/or marker for detecting sumoylation of Tcf-4 is introduced), and SUMO (to which the signal and/or marker is introduced), and
(D): cells that have co-expressed PIAS, SUMO, and Tcf-4.
   A still further aspect of the present invention relates to a method according to claim 3 for identifying a compound that inhibits the sumoylation of Tcf-4 by PIAS, comprising the steps of: (i) using the cells described in (E) or (F), below, and contacting the cells with a test compound, and (ii) determining whether the test compound inhibits the sumoylation of Tcf-4 by PIAS by measuring a fluorescent intensity of the green fluorescent protein (GFP) after adding a luciferase substrate, wherein cells (E) and (F) are as follows:
(E): cells that have co-expressed a fused protein of Tcf-4 and GFP, a fused protein of SUMO and luciferase, and PIAS; and
(F): cells that have co-expressed a fused protein of Tcf-4 and luciferase, a fused protein of SUMO and GFP, and PIAS.

A further aspect of the present invention relates to a method according to claim 4 for identifying a compound that inhibits the sumoylation of Tcf-4 by PIAS, comprising the steps of: (i) bringing PIAS, E1 SUMO-activating enzyme, E2 SUMO-conjugation enzyme, SUMO or SUMO to which a signal and/or marker for detecting sumoylation of Tcf-4 is introduced, and a test compound, to contact with Tcf-4 or Tcf-4 immobilized on a solid phase, and (ii) determining whether the test compound inhibits the sumoylation of Tcf-4 by PIAS, by detecting the sumoylated Tcf-4, or by detecting the presence, absence, or alteration of the signal and/or marker.

A further aspect of the present invention relates to a method according to claim 5 for identifying a compound that inhibits the sumoylation of Tcf-4 by PIAS, comprising the steps of: (i) bringing PIAS, E1 SUMO-activating enzyme, E2 SUMO-conjugation enzyme, SUMO labeled with a radioactive isotope, biotin, or a peptide-tag, and a test compound to contact with Tcf-4 immobilized to a solid phase; and (ii) determining whether the test compound inhibits the sumoylation of Tcf-4 by PIAS by measuring the substance used for labeling.

A still further aspect of the present invention relates to a method according to claim 6 for identifying a compound that inhibits the sumoylation of Tcf-4 by PIAS, comprising the steps of: (i) bringing PIAS, E1 SUMO-activating enzyme, E2 SUMO-conjugation enzyme, SUMO (labeled with europium), Tcf-4 (labeled with a substance radiating secondary fluorescence upon excitation by fluorescence irradiated by europium), and a test compound to reaction; and (ii) determining whether the test compound inhibits the sumoylation of Tcf-4 by PIAS by irradiating light of a wavelength that excites europium and then measuring the secondary fluorescence intensity.

A further aspect of the present invention relates to a method according to claim 7 for identifying a compound that inhibits the sumoylation of Tcf-4 by PIAS, comprising the steps of: (i) bringing PIAS, E1 SUMO-activating enzyme, E2 SUMO-conjugation enzyme, and a test compound to react with one of the following: a fused protein of Tcf-4 and luciferase as well as a fused protein of SUMO and green fluorescent protein (GFP), or a fused protein of Tcf-4 and GFP as well as a fused protein of SUMO and luciferase, and (ii) determining whether the test compound inhibits the sumoylation of Tcf-4 by PIAS by measuring the fluorescence intensity of the GFP after adding a luciferase substrate.

### BRIEF DESCRIPTION OF THE DRAWINGS

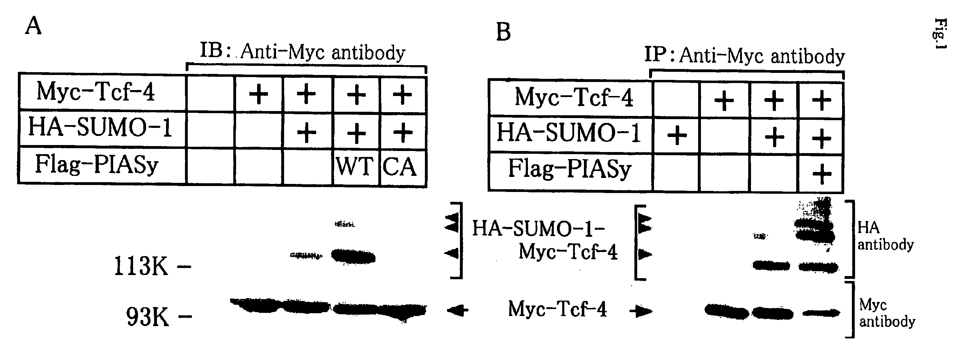

FIG. 1A and FIG. 1B illustrate that the conjugation of SUMO-1 to Tcf-4 was accelerated by PIASy. In FIG. 1A, conjugation of SUMO-1 to TCF-4 was carried out using cells in which their respective genes had been transfected and was detected by Western Blotting using an anti-Myc antibody. In the figure, "WT" refers to a wild-type PIASy, and "CA" refers to a PIASy wherein cysteines (C) at positions 342 and 347 in the amino acid sequence of PIASy were substituted by alanines (A). In FIG. 1B, conjugation of SUMO-1 to TCF-4 was carried out using cells in which the respective genes had been transfected. After lysis of the cells, Myc-Tcf-4 was immunoprecipitated with an anti-Myc antibody, and the recovered protein was detected by Western Blotting using an anti-Myc antibody and an anti-HA antibody.
FIG. 2 illustrates that PIASy by itself accelerates the transcriptional activity of Tcf-4, and also promotes the β-catenin- or Wnt-3a-dependent transcriptional activity of Tcf-4. The transcriptional activity of Tcf-4 was measured using a firefly luciferase gene as a reporter gene. In the figure, the longitudinal axis indicates relative luciferase induction, expressed as a fold increase of the luciferase activity. The values in the figure are the amount of each plasmid added (µg).
FIG. 3 illustrates that SUMO-1 accelerates the β-catenin-dependent transcriptional activity of Tcf-4. The values in the figure are the amounts of the respective plasmids added (µg).
FIG. 4 illustrates that acceleration of the β-catenin-dependent transcriptional activity of Tcf-4 is inhibited by Axam, which has desumoylation activity, but is not inhibited by an Axam mutant that does not have desumoylation activity. In the figure, "WT" refers to wild-type Axam, and "CS" refers to an Axam mutant in which cysteine (C) at position 547 in the amino acid sequence was substituted by serine (S), and "72-588", "72-400", and "381-588" refer to deletion-type Axam mutants.
FIG. 5 is a schematic diagram showing the structures of human PIASy, human Tcf-4, Axam, and various mutants of Axam.
FIG. 6 illustrates that PIASy accelerates the conjugation of SUMO-1 to Tcf-4 in vitro, but that PIASyCA does not conjugate SUMO-1 to Tcf-4. The values shown on the left side represent the molecular weights.
FIG. 7 illustrates that any of PIASy, PIAS1, PIAS3, and PIASxα accelerates the conjugation of SUMO-1 to Tcf-4.

### DETAILED DESCRIPTION OF INVENTION

The present invention claims the benefit of priority of Japanese Patent Application No. 2002-55796 and No. 2002-271527.

Technical and scientific terms used herein have the meanings as understood generally by those skilled in the art, unless otherwise defined. Reference is made herein to a variety of methods that are well-known to those skilled in the art.

A mode of embodiment of the present invention may be described in more detail hereafter. The following detailed description is illustrative and merely explanatory.

In the present invention, it has been found that the sumoylation of transcriptional factor Tcf-4 is accelerated by PIASy, and that the sumoylation enhances the Tcf-4 function. Tcf-4 is a DNA-binding protein belonging to the Tcf family. Tcf-4 is located downstream of the Wnt signaling pathway, and acts as a transcriptional factor by binding to β-catenin. β-catenin-dependent enhancement of the transcriptional activity of Tcf-4 was increased synergistically by PIASy. Wnt signal-dependent enhancement of the transcriptional activity of Tcf-4 was also increased synergistically by PIASy, where the Wnt signal shows the enhancing effect on the signal by suppressing the degradation of β-catenin. In addition, it has been found that the β-catenin-dependent enhancement of the transcriptional activity of Tcf-4 was inhibited by an Axam protein with desumoylation activity (non-patent document 7 and non-patent document 8), which revealed that the enhancement of the Tcf-4 function results from sumoylation. Although these results were obtained by use of PIASy, it is believed that the transcriptional activity of Tcf-4 can be similarly enhanced by PIAS1, PIAS3, and PIASxα, since PIAS1, PIAS3, and PIASxα also provide sumoylation to Tcf-4.

Therefore, it is found that an enhanced function of Tcf-4, e.g. its transcriptional activity, can be downregulated by inhibiting Tcf-4 sumoylation and/or by desumoylating sumoylated Tcf-4. A signal transduction system via β-catenin/Tcf-4 is believed to be involved in cell proliferation. Accordingly, suppression of Tcf-4-associated cell proliferation can be achieved by inhibiting Tcf-4 sumoylation and/or by desumoylation of sumoylated Tcf-4.

Tcf-4 is expressed in normal as well as neoplastic intestine epithelial tissues, and in all colon cancer cell lines examined (non-patent document 11). Further, the following have been reported: that transcriptional activity due to the β-catenin/Tcf complex is constantly activated in many cases of colon cancer (non-patent document 11); that a β-catenin/Tcf signal is activated by mutation of the β-catenin or tumor suppressor gene APC (Adenomatous poliposis coli)(non-patent document 12 and non-patent document 13) in colon cancer; and that in colon cancer cells, β-catenin regulates the expression of cyclin D1, which is associated with cell cycle progression (non-patent document 14).

Since there seems to be a close relationship between enhancement of transcriptional activity in the β-catenin/Tcf signal transduction system and colon cancer, it is believed that colon cancer can be treated by means of inhibiting the transcriptional activity of Tcf-4 by inhibiting Tcf-4 sumoylation and/or by desumoylating sumoylated Tcf-4.

In the present invention, the term "PIAS" refers to a protein belonging to the PIAS family proteins. In the present invention, the E3 ligase that associates with Tcf-4 sumoylation may be any PIAS, preferably PIASy, PIAS1, PIAS3, or PIASxα, and more preferably PIASy. Also, two or more PIAS proteins may be used as the E3 ligase.

In the present invention, the term "SUMO" includes all members of the SUMO family, such as SUMO-1, SUMO-2, and/or SUMO-3.

Herein, the term "inhibitor of transcriptional activity of Tcf-4" refers to a substance that ultimately inhibits Tcf-4-associated transcriptional activity by acting in some manner on a transcriptional activation apparatus including Tcf-4, where the apparatus comprises, e.g., binding of Tcf-4 to a specific DNA sequence or binding of Tcf-4 to another factor that carries out transcription in association with Tcf-4.

According to the present invention, based on the above information, there is provided a method for identifying a compound that inhibits the sumoylation of Tcf-4 by PIAS. A compound that inhibits the sumoylation of Tcf-4 by PIAS can be identified by selecting conditions allowing for the interaction of PIAS and/or Tcf-4 with a test compound, contacting PIAS and/or Tcf-4 with the test compound under such conditions, and then detecting the sumoylation of Tcf-4.

Detection of Tcf-4 sumoylation can be conducted by introducing into the above identification method a system that uses a signal and/or marker capable of detecting Tcf-4 sumoylation, and then detecting the signal and/or marker. Such signal and/or marker may be used by introducing the signal and/or marker to SUMO and/or Tcf-4. Regarding SUMO, any SUMO may be used as long as it belongs to the SUMO family. SUMO-1 is preferably used. The term "signal" as used herein refers to a substance that is directly detectable by its physical or chemical properties, and the term "marker" refers to a substance that is indirectly detectable when its physical or biological properties are used as an indicator. Examples of a signal include luciferase, green fluorescein protein (hereinafter, abbreviated as GFP), europium, and a radioactive isotope (¹²⁵I, ³H, ¹⁴C, ³⁵S, etc). Examples of a marker include a reporter gene (such as the chloramphenicol acetyltransferase (CAT) gene), a tag for detection (such as 6xHis-tag, Myc-tag, EE-tag, Express-tag, V5-tag), or a known substance such as biotin. A signal or marker is not limited to the above examples, and any substance may be used as long as it makes it possible to detect the sumoylation of Tcf-4 by PIAS. Methods for detecting such signals or markers are well known to those skilled in the art.

Sumoylated Tcf-4 may be also detected using, e.g., an anti-Tcf-4 antibody. When Tcf-4 has been previously modified with another protein or peptide, sumoylated Tcf-4 can be detected using an antibody for the protein or peptide in question. Sumoylation of Tcf-4 is detectable, e.g., by detecting the difference in molecular weight between sumoylated Tcf-4 and non-sumoylated Tcf-4 utilizing variations in their respective mobilities in electrophoresis, using a known protein detection method such as Western Blotting.

Sumoylation of Tcf-4 may also be measured using the transcriptional activity of Tcf-4 as an indicator, since the sumoylation of Tcf-4 enhances the transcriptional activity thereof. The transcriptional activity of Tcf-4 can be measured by a known method for measuring the activity of a transcriptional factor using a reporter gene, e.g. a reporter plasmid having DNA with a Tcf-recognizable nucleotide sequence in its upstream (hereinafter, also referred to as a "Tcf-responsive type plasmid").

A compound inhibiting the sumoylation of Tcf-4 can be obtained according to the above identification method by comparing an amount of Tcf-4 sumoylated under conditions in which a test compound is not present with an amount of Tcf-4 sumoylated under conditions in which the test compound is present, and selecting the test compound with respect to the amount that decreases. Such comparison is carried out by detecting the presence, absence, or alteration of a signal and/or marker capable of detecting sumoylation of Tcf-4. Alternatively, such a comparison can be conducted by measuring the difference in molecular weight between sumoylated Tcf-4 and non-sumoylated Tcf-4 using a known method, such as Western Blotting.

The identification method can be carried out utilizing a well-known pharmaceutical screening system. PIAS, Tcf-4, and SUMO used in the identification method may be the following: products in cells that have expressed PIAS, Tcf-4, and SUMO by genetic engineering techniques; synthetic products in a cell-free system; chemically synthesized products; products prepared from cells or from any biological samples; or products that are further purified from the foregoing. Further, as long as sumoylation of Tcf-4 by PIAS is not inhibited, PIAS, Tcf-4, and SUMO may be those to which another protein or peptide (such as glutathione S-transferase (GST), luciferase, GFP, β-galactosidase, Fc fragments of immunoglobulin such as IgG, His-tag, Myc-tag, EE-tag, Express-tag, V5-tag, or Flag-tag) is ligated directly or indirectly via a linker peptide to the N-terminus side or C-terminus side of PIAS, Tcf-4, or SUMO by using genetic engineering techniques. Examples of test compounds include compounds derived from chemical libraries or natural products, or compounds obtained by drug designing based on the three-dimensional structures of PIAS and Tcf-4.

Any of the following systems can be used as the above identification method: a system where PIAS, Tcf-4, and SUMO are expressed in cells by genetic engineering techniques to induce sumoylation in the cells (in situ system), or a system where PIAS, Tcf-4, and SUMO are employed to induce sumoylation in vitro. In order to induce sumoylation in vitro, further addition of an E1 SUMO-activating enzyme and/or an E2 SUMO-conjugation enzyme other than PIAS, Tcf-4 and SUMO is preferred.

An example of the in situ system includes a system that uses the cells described in (A), (B), (C), or (D) below, brings the cells to contact with a compound, and then detects the sumoylated Tcf-4, or detects the presence, absence, or alteration of a signal and/or marker for detecting Tcf-4 sumoylation, wherein cells (A), (B), (C), and (D) are as follows:
(A): cells that have co-expressed PIAS, SUMO, and Tcf-4 to which a signal and/or marker for detecting sumoylation of Tcf-4 is introduced;
(B): cells that have co-expressed PIAS, Tcf-4, and SUMO to which a signal and/or marker for detecting sumoylation of Tcf-4 is introduced;
(C): cells that have co-expressed PIAS, Tcf-4 to which a signal and/or marker for detecting sumoylation of Tcf-4 is introduced, and SUMO to which the signal and/or marker is introduced; and
(D): cells that have co-expressed PIAS, SUMO, and Tcf-4.

For the above cells, cells that are generally used for gene transfection can be preferably used. More preferably used are animal cells, and even more preferably used are human colon cancer cells.

As a specific system thereof, a compound that inhibits the sumoylation of Tcf-4 by PIAS can be identified by the following method: providing cells that have expressed PIAS, Myc-tagged Tcf-4, and SUMO; contacting the cells with a test compound; conducting electrophoresis after a common pretreatment of the cells; and detecting bands corresponding to the sumoylated Tcf-4 by Western Blotting using an anti-Myc antibody (see Example 1).

It is also possible to identify a compound that inhibits the sumoylation of Tcf-4 by PIAS as well as the transcriptional activity of Tcf-4, by the following method: providing cells that have expressed HA-tagged Tcf-4, PIAS, SUMO, and β-catenin, through a Tcf-responsive type plasmid; contacting the cells with a test compound; and detecting a signal and/or marker derived from the Tcf-response type plasmid (see Example 2).

In the case of a method using Renilla luciferase and GFP, cells that constitutively express a fused protein of GFP and Tcf-4 (GFP-Tcf-4) as well as a fused protein of Renilla luciferase and SUMO (Renilla-SUMO) are employed. When a Renilla luciferase substrate is added to cells in a state where Renilla-SUMO has been conjugated to GFP-Tcf-4 by sumoylation by PIAS, light generated by the luminous reaction of the Renilla luciferase irradiates onto the Tcf-4-linked GFP, whereby the GFP emits secondary fluorescence. Measurement of the secondary fluorescence intensity enables the quantification of Tcf-4 sumoylation in the cells. Thus, by contacting the cells with a test compound, and then measuring the fluorescence intensity of GFP after a specified period of time, it is possible to determine the level of Tcf-4 sumoylation. This method is highly useful, since it is possible to prevent an increase in the existing background in a GFP-Tcf-4/Renilla-SUMO system by applying, e.g., an ecdyson-inducing expression system so as to control Renilla-SUMO expression. This method, using a combination of a fused protein of Tcf-4 and Renilla luciferase (Renilla-Tcf-4) as well as a fused protein of SUMO and GFP (GFP-SUMO), may be used in a similar manner to the method described above. The luciferase is not limited to Renilla luciferase, and any known luciferase may be used. In these identification methods, the combination of substances to be fused with Tcf-4 and SUMO is not limited to GFP and Renilla luciferase, and any combination of substances may be used as long as a signal emitted by one of the substances, for example, fluorescence or the like, acts on the other substance to cause the emission of a signal that is different from the initial signal, such as, for example, fluorescence of a different wavelength.

An example of the in vitro system includes a system in which PIAS, E1 SUMO-activating enzyme, E2 SUMO-conjugation enzyme, SUMO or SUMO to which a signal and/or marker for detecting sumoylation of Tcf-4 is introduced, and a test compound, are contacted with Tcf-4 or Tcf-4 immobilized to a solid phase, and sumoylated Tcf-4 is then detected, or the presence, absence, or alteration of the signal and/or marker is detected. PIAS, Tcf-4, SUMO, E1 SUMO-activating enzyme, and E2 SUMO-conjugation enzyme may be the following: synthetic products in a cell-free system; chemically synthesized products; products prepared from cells which have expressed those products by genetic engineering techniques, products from any biological samples; or products that are further purified from the foregoing.

Examples of a signal and/or marker to be introduced to SUMO include a radioactive isotope, biotin, peptide tag, and the like. Radioactive isotopes that can be used herein include ¹²⁵I, ³H, ¹⁴C, and ³⁵S. Peptide tags that can be used herein include Flag-tag, HA-tag, Myc-tag, EE-tag, Express-tag, and V5-tag. Introduction of these signals and/or markers to SUMO can be performed by a known method.

Detection of the above signal and/or marker is possible by a conventional method. A radioactive isotope, for example, may be detected by measuring the radioactivity with a commercially available measuring apparatus. Biotin can be detected by, e.g., binding biotin to streptavidin that has been labeled with alkaline phosphatase (AP) or horseradish peroxidase (HRP), adding a substrate for the AP or the HRP, and measuring the enzymatic activity. A peptide tag can be detected by using an antibody to the peptide tag that is labeled with AP or HRP, binding the antibody to the peptide tag, adding a substrate for the AP or the HRP, and measuring the enzymatic activity. As the substrate for AP or HRP, a substrate that is generally known to those skilled in the art may be used.

Examples of a solid phase that may be used herein include, but are not limited to, a commercially available multi-well plate, agarose or Sepharose beads, or SPA beads. Tcf-4 can be immobilized onto a solid phase by a conventional method. For example, when using a fused protein of Tcf-4 and His-tag, a solid phase pre-coated with nickel ion is used, and then Tcf-4 is immobilized on the solid phase. When using a fused protein of Tcf-4 and GST, glutathione is used for the coating of the solid phase. It is also acceptable to use a commercially available glutathione-coated multi-well plate or beads. In cases where Tcf-4 is used without being fused with a protein or peptide, or where immobilization to a solid phase is not performed via a protein or peptide fused with Tcf-4, Tcf-4 can be immobilized on the solid phase by pre-coating the solid phase with an anti-Tcf-4 antibody, or by using commercially available agarose beads or Sepharose beads that are pre-coated with an anti-Tcf-4 antibody.

As specific methods, methods that use SPA beads, glutathione-Sepharose beads, and a multi-well plate are described below. However, the present invention is not limited to these methods.

In a method using SPA beads, human PIAS, Ubc9, Aosl/Uba2, GST-fused Tcf-4, and SUMO are expressed, e.g., by genetic engineering techniques (Escherichia coli or infection of Baculovirus to insect cells), purified, and used. SUMO is labeled with a radioactive isotope, e.g., ¹²⁵I. These purified proteins and a test compound are mixed, to conduct sumoylation of Tcf-4. Glutathione-coated SPA beads (Amersham Corp.) are added to the reaction solution, and the solution is allowed to stand. During this period, the GST-Tcf-4 binds to the glutathione-coated SPA beads, and the ¹²⁵I-labeled SUMO then binds to the GST-Tcf-4, whereby the SPA beads are irradiated by radiation emitted by ¹²⁵I, resulting in the emission of scintillation florescence. By measuring this fluorescence, the amount of Tcf-4 sumoylation can be quantified. Since the inhibition of sumoylation would lower the measured values, this method enables the detection of a compound that inhibits sumoylation.

In a method using glutathione-Sepharose beads, human PIAS, Ubc9, Aosl/Uba2, GST-fused Tcf-4, and SUMO are expressed, e.g., by genetic engineering techniques (Escherichia coli or infection of Baculovirus to insect cells), purified, and used. SUMO is labeled with a radioactive isotope, e.g., ¹²⁵I. These purified proteins and a test compound are mixed, to conduct sumoylation of Tcf-4. Glutathione-Sepharose beads (Amersham Corp.) are added to the reaction solution. After stirring, the beads are washed, to collect the beads to which GST-Tcf-4 has bound. A liquid scintillator is then added to the collected glutathione-Sepharose beads to measure the amount of ¹²⁵I-SUMO conjugated to Tcf-4. Since inhibition of sumoylation by a test compound would lower the radioactivity in comparison with a case in which the test compound is not used, this method enables the detection of a compound that inhibits sumoylation.

In a method using a multi-well plate, human PIAS, E1 SUMO-activating enzyme, E2 SUMO-conjugation enzyme, SUMO, and a fused protein of Tcf-4 and His-tag (His-Tcf-4) are expressed, e.g., by genetic engineering techniques (Escherichia coli or infection of Baculovirus to insect cells), purified, and used. SUMO is labeled with a radioactive isotope, e.g., ¹²⁵I. His-Tcf-4 is immobilized on, e.g., a Ni⁺-coated 96- or 384-well plate. A test compound, E1 SUMO-activating enzyme, E2 SUMO-conjugation enzyme, ¹²⁵I-SUMO, and PIAS are added to each well, and a sumoylation reaction is performed. After the reaction, the plate is washed, and the amount of ¹²⁵I-SUMO conjugated to His-Tcf-4 is measured. Since inhibition of sumoylation by using a test compound would lower the radioactivity in comparison with a case in which the test compound is not used, this method enables the detection of a compound that inhibits sumoylation.

An example of another in vitro system that can be used herein is a system using the proteins described in (a) or (b) below, wherein the proteins are contacted with a test compound, followed by detection of the presence, absence, or alteration of a signal and/or marker for detecting sumoylation of Tcf-4, wherein proteins (a) and (b) are as follows:
(a): PIAS, E1 SUMO-activating enzyme, E2 SUMO-conjugation enzyme, SUMO (to which a signal and/or marker for detecting sumoylation of Tcf-4 is introduced), and Tcf-4 (to which a signal and/or marker for detecting the signal and/or marker introduced to SUMO is introduced); and
(b): PIAS, E1 SUMO-activating enzyme, E2 SUMO-conjugation enzyme, Tcf-4 (to which a signal and/or marker for detecting sumoylation of Tcf-4 is introduced), and SUMO (to which a signal and/or marker for detecting the signal and/or marker introduced to Tcf-4 is introduced).

As a specific method, a method using europium-labeled SUMO (europium-SUMO), and a fused protein of Tcf-4 and XL665 (XL665-Tcf-4) is exemplified. Human PIAS, E1 SUMO-activating enzyme, E2 SUMO-conjugation enzyme, XL665-Tcf-4, and SUMO are expressed, e.g., by genetic engineering techniques (Escherichia coli or infection of Baculovirus to insect cells), purified, and used. SUMO is labeled with europium by a conventional method. A test compound, XL665-Tcf, E1 SUMO-activating enzyme, E2 SUMO-conjugation enzyme, europium-SUMO, and PIAS are added to each well, and a sumoylation reaction is carried out. After the reaction, light of 337 nm is irradiated into the wells so as to induce fluorescence emission from europium, and then a secondary fluorescence (665 nm), which is emitted from XL665 as a result of the exposure to the europium fluorescence, is measured and the amount of europium-SUMO conjugated to XL665-Tcf-4 is determined. Since inhibition of sumoylation by the test compound would lower the secondary fluorescence intensity in comparison with a case in which the test compound is not used, this method enables the detection of a compound that inhibits sumoylation.

As another specific method, a method using a fused protein of Tcf-4 and Renilla luciferase (Renilla-Tcf-4) as well as a fused protein of SUMO and GFP (GFP-SUMO) is exemplified. Human PIAS, E1 SUMO-activating enzyme, E2 SUMO-conjugation enzyme, Renilla-Tcf-4, and GFP-SUMO are expressed, e.g., by genetic engineering techniques (Escherichia coli or infection of Baculovirus to insect cells), purified, and used. A test compound, Renilla-Tcf-4, E1 SUMO-activating enzyme, E2 SUMO-conjugation enzyme, GFP-SUMO, and PIAS are added to each well of a plate, and a sumoylation reaction is carried out. After the reaction, a luciferase substrate is added to the well to induce a luminous reaction, and the fluorescence from the excited GFP that results from the luminous reaction is measured, to allow detection of the amount of SUMO conjugated to Tcf-4. Since inhibition of sumoylation by the test compound would lower the luminescent intensity in comparison with a case in which the test compound is not used, this method enables the detection of a compound that inhibits sumoylation.

A method using a combination of a fused protein of Tcf-4 and GFP (GFP-Tcf-4) as well as a fused protein of SUMO and Renilla luciferase (Renilla-SUMO) may be also applied similarly to the above method.

Each of the specific examples of identification methods described above illustrates an aspect of the present identification method, and the present identification method is not limited to these examples. Any method that can detect the conjugation of SUMO to Tcf-4 resulting from an interaction of PIAS with Tcf-4 is included in the scope of the present invention.

A compound obtained by the above method can be used as an inhibitor of sumoylation of Tcf-4 by PIAS, and an inhibitor of transcriptional activity of Tcf-4 by inhibiting the sumoylation of Tcf-4 by PIAS. Examples of such a compound include a compound that inhibits the interaction of PIAS with Tcf-4, and a compound that inhibits the activity of PIAS for Tcf-4 sumoylation. For example, a peptide or oligopeptide comprising an amino acid sequence of a region in which the two proteins interact with each other may be exemplified. The peptide or oligopeptide can be identified by designing the peptide or oligopeptide on the basis of the amino acid sequence of PIAS or Tcf-4, synthesizing the peptide or oligopeptide by a known peptide synthesizing method, and testing the peptide or oligopeptide according to the above method to determine whether the thus-obtained peptide or oligopeptide inhibits the binding of PIAS and Tcf-4 as well as the sumoylation of Tcf-4 by PIAS. Further, an antibody that inhibits the interaction of PIAS with Tcf-4 may be exemplified as one of the above compounds. The antibody can be obtained, for example, using a peptide or oligopeptide (comprising an amino acid sequence of a region in which the two proteins interact with each other) as an antigen, by using antibody preparation methods well-known in the art.

A compound inhibiting the expression and/or function of PIAS is also included in the scope of the present invention, since the sumoylation of Tcf-4 by PIAS may be inhibited as a result thereof. Identification of a compound that inhibits PIAS expression can be performed using a well-known screening system for inhibitors of protein expression. As an example of a compound that inhibits the expression of PIAS, an antisense oligonucleotide of the PIAS gene may be exemplified. The antisense oligonucleotide can be obtained by selecting an oligonucleotide that specifically inhibits the expression of the PIAS gene, from oligonucleotides designed on the basis of the nucleotide sequence of the PIAS gene, using an expression system of the PIAS gene. Identification of a compound that inhibits PIAS function may be performed by the above identification method using PIAS, by selecting a substance that inhibits a function thereof, such as sumoylation activity.

It has been clarified in the present invention that sumoylated Tcf-4 can have an enhanced transcriptional activity, and therefore it is further expected that a desumoylation agent, which releases SUMO from sumoylated Tcf-4, is useful for suppression of the transcriptional activity of Tcf-4 as well as for prevention and/or treatment of a disease that depends on enhanced transcriptional activity of Tcf-4, such as colon cancer. Examples of a desumoylation agent include Axam, which has desumoylation activity and which will be described hereafter in the Examples, or a deletion mutant thereof that retains the same activity.

The thus selected compound, the inhibitor of Tcf-4 sumoylation by PIAS, the desumoylation agent for sumoylated Tcf-4, and the inhibitor of transcriptional activity of Tcf-4 comprising the inhibitor and/or the desumoylation agent, may be used as a reagent by using it alone or in combination. Such a reagent is useful for research concerning, e.g., sumoylation or a β-catenin/Tcf-4 signal transduction system.

The compounds selected above, the inhibitor of Tcf-4 sumoylation by PIAS, the desumoylation agent of sumoylated Tcf-4, and the inhibitor of transcriptional activity comprising the inhibitor and/or the desumoylation agent, can be formulated as a medicinal composition upon selection after consideration of the balance between biological usefulness and toxicity. Such compounds, an inhibitor of Tcf-4 sumoylation, a desumoylation agent for Tcf-4, and an inhibitor of transcriptional activity of Tcf-4, may be used alone or in combination.

The above compounds, the inhibitor of Tcf-4 sumoylation, the desumoylation agent for Tcf-4, the inhibitor of transcriptional activity of Tcf-4, and the medicinal composition can be applied to prevention and/or treatment of a cell proliferation-induced disease such as cancer. Furthermore, since it has been reported that Tcf-4 is activated in colon cancer, the above medicinal composition is useful for prevention and/or treatment of colon cancer.

In terms of the formulation, the inhibitor of Tcf-4 sumoylation by PIAS, the desumoylation agent of sumoylated Tcf-4, the inhibitor of transcriptional activity comprising the inhibitor and/or the desumoylation agent, and the medicinal composition, are preferably formulated in combination with suitable pharmaceutical carriers. Such a formulation comprises a therapeutically effective amount of the above compounds, the above inhibitor of sumoylation, the above desumoylation agent, the above inhibitor of transcriptional activity, and/or the above medicinal composition, as well as a pharmaceutically acceptable carrier or vehicle. Examples of a carrier include physiological saline, buffered physiological saline, dextrose, water, glycerol, ethanol, and a mixture thereof. However, it is not limited thereto. The formulation may be selected according to an administration route, and such formulation is well-known to those skilled in the art.

The above inhibitor of sumoylation, the above inhibitor of transcriptional activity, the above desumoylation agent, and the above medicinal composition can be used alone or in combination with other compounds that are therapeutically effective.

In terms of the mode of administration, it may be either systemic administration or local administration. One preferred mode of systemic administration is injection, e.g., an intravenous injection. Other injection routes, such as subcutaneous, intramuscular, or intraperitoneal injection, may also be used. Another mode of administration may be oral administration, so long as an enteric formulation or a capsule formulation can be suitably formulated. In addition, transmucosal administration or percutaneous administration, which comprises using a penetrant such as bile salt, fusidic acid, or other surfactants, may also be used. In a local administration, forms such as salve, paste, or gel, may be used.

Suitable dosage ranges can be determined according to the following: the effectiveness of the above inhibitor of sumoylation, the above desumoylation agent, the above inhibitor of transcriptional activity, and the above medicinal composition; the administration route thereof; the characteristics of the formulation; the symptomatic conditions of a subject; and the judgment of a doctor in attendance. Specifically, a suitable dosage may fall, e.g., within a range of 0.1 to 100 µg per 1 kg of the body weight of a subject. However, the dosage may be altered by means of common conventional experiments for optimization of a dosage that are well-known in the art.

Preparation of a pharmaceutical may be carried out by well-known formulating procedures in accordance with the physical properties of the substance employed, for example, peptides, proteins, oligonucleotides, antibodies, or compounds. Specifically, formulations such as powdered drugs, pills, tablets, capsules, aqueous solutions, liposomal formulations, fat emulsions, clathrates (such as those of cyclodextrin), and the like can be used.

Powdered drugs, pills, capsules, or tablets can be prepared using, for example, an excipient such as lactose, glucose, sucrose, or mannitol; a disintegrant such as starch or sodium arginate; a lubricant such as magnesium stearate or talc; a binder such as polyvinylalcohol, hydroxypropyl cellulose, or gelatin; a surfactant such as fatty acid ester; and a plasticizer such as glycerin, or the like. For preparation of a tablet or capsule, a solid pharmaceutical carrier is used.

A suspension can be prepared using water; saccharides such as sucrose, sorbitol, or fructose; glycols such as PEG; and oils.

Injectable solutions can be prepared using a carrier comprising a saline solution, a glucose solution, or a mixture of the salt water and glucose solution.

Inclusion into a liposome formulation may be conducted in the following manner: by dissolving the substance of interest in a solvent (e.g., ethanol) to make a solution, adding a solution of phospholipids dissolved in an organic solvent (e.g., chloroform), removing the solvent by evaporation and adding a phosphate buffer thereto, agitating the solution and then subjecting it to sonication followed by centrifugation to obtain supernatant, and finally, filtrating the supernatant for recovering a liposome.

Fat emulsion can be prepared in the following manner: by mixing the substance of interest, an oil ingredient (vegetable oil such as soybean oil, sesame oil, olive oil, or MCT), an emulsifier (such as phospholipid), and the like; heating the mixture to make a solution; adding water of a required quantity; and then emulsifying or homogenizing by use of an emulsifier (homogenizer, e.g., a high pressure jet type, an ultrasonic type, or the like). The fat emulsion may be also lyophilized. For conducting lipid-emulsification, an auxiliary emulsifier may be added, and examples thereof include glycerin or saccharides (e.g., glucose, sorbitol, fructose, etc.).

Inclusion into a cyclodextrin formulation may be carried out in the following manner: by dissolving the substance of interest in a solvent (e.g., ethanol); adding a solution of cyclodextrin dissolved in water under heating thereto; chilling the solution and filtering the precipitates; and drying under sterilization. At this time, the cyclodextrin to be used may be appropriately selected from among those having different void sizes (α, β, or γ type) in accordance with the bulkiness of the substance.

The present invention also provides a reagent kit, comprising at least the following: PIAS and/or Tcf-4; a polynucleotide encoding PIAS and/or a polynucleotide encoding Tcf-4; or a vector comprising a polynucleotide encoding PIAS and/or a vector comprising a polynucleotide encoding Tcf-4. The reagent kit can be used in the above identification methods. PIAS and Tcf-4 may be any of the following: products in cells that have expressed PIAS or Tcf-4 by genetic engineering techniques; synthesized products in a cell-free system; chemically synthesized products; products prepared from the cells or any biological samples; or products that are further purified from the foregoing. Further, as long as sumoylation of Tcf-4 by PIAS is not inhibited, Tcf-4 and PIAS may be those to which another protein or peptide (such as glutathione S-transferase (GST), luciferase, green fluorescent protein (GFP), β-galactosidase, immunoglobulin Fc fragments like IgG, His-tag, Myc-tag, or Flag-tag) is ligated directly or indirectly via, e.g., a linker peptide to the N-terminus side or C-terminus side of PIAS or Tcf-4 by, e.g., genetic engineering techniques. A polynucleotide encoding PIAS or Tcf-4 may be prepared from a human cDNA library by known genetic engineering techniques. A vector comprising a polynucleotide that encodes PIAS or Tcf-4 is obtained by inserting the above polynucleotide into a suitable expression vector DNA (such as a vector derived from a bacterial plasmid) by known genetic engineering techniques. The reagent kit may further comprise materials necessary for the above identification methods; a signal and/or marker for detecting the interaction of PIAS with Tcf-4, such as Tcf-4 sumoylation by PIAS; a signal and/or marker for detecting the enhanced function of Tcf-4 by PIAS; SUMO, or another protein- or peptide-modified SUMO; a polynucleotide encoding SUMO; a vector carrying the polynucleotide; or a buffer and the like. In terms of preparation thereof, it is sufficient to use a well-known means for the preparation suitable for each substance to be used.

### Examples

The present invention may be described more particularly in the following examples, but the present invention is not limited to these examples.

Plasmids used in the following examples were constructed first.

A cDNA coding for human Tcf-4 having a Myc-tag was inserted into a protein expression vector for mammalian cells, to construct a Myc-Tcf-4 expression plasmid.

A cDNA coding SUMO was inserted into a protein expression vector with an HA-tag sequence for mammalian cells, to construct an HA-SUMO expression plasmid.

A cDNA coding PIASy having a Flag-tag was inserted into a protein expression vector with a cytomegalovirus promoter for mammalian cells, to construct a Flag-PIASy expression plasmid. A plasmid containing mutated cDNA for substitution of cysteines (C) at positions 342 and 347 in the amino acid sequence of PIASy with alanines (A) (PIASyCA) was also constructed.

A cDNA coding for β-catenin (whose GSK-3β phosphorylation site at the N-terminus was substituted with alanine, and which had an HA-tag) was inserted into a vector pUC19-EF1α at the KpnI/SalI site thereof, to construct an HA-β-catenineSA expression plasmid.

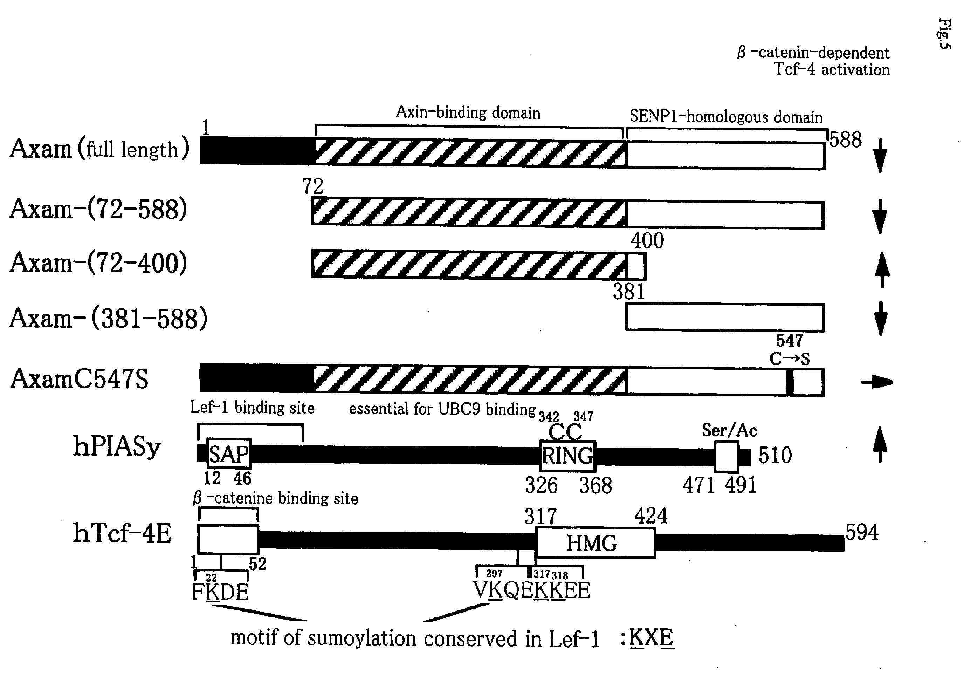

cDNAs (FIG. 5) coding for Axam (non-patent document 8) or the deletion mutants thereof were inserted into protein expression vectors for mammalian cells, to construct their respective expression plasmids.

### Example 1

### (Detection of SUMO-1 conjugation to Tcf-4)

Human embryonic kidney 293 cells (hereinafter, abbreviated as HEK293 cell) were seeded in the amount of 3 × 10⁵ cells in a 35 mm-diameter dish, and incubated for 24 h. After incubation, the cells were transfected with 1 µg of the Myc-Tcf-4 expression plasmid, 0.25 µg of the HA-SUMO-1 expression plasmid, and 0.02 µg of the Flag-PIASy expression plasmid according to the combinations shown in FIGs. 1A and 1B, using LipofectAMINE 2000 manufactured by GIBCO BRL Corp. Gene transfection was similarly performed using the Flag-PIASyCA expression plasmid in place of the Flag-PIASy expression plasmid. Transfection was performed according to the manufacturer's instructions. After the gene transfection, cells were incubated for 40 h, followed by treatment with 10% trichloroacetic acid (TCA) containing 2 mM dithiothreitol (DTT), to give a sample for electrophoresis. Tcf-4 with Myc-tag that was expressed in the cells was detected by Western Blotting using an anti-Myc antibody (FIG. 1A).

The HEK 293 cells, which were transfected with each gene by the same manner as described above to express proteins, were lysed by adding RIPA buffer (10 mM Na-phosphate buffer, pH 7.2, 150 mM NaCl, 1% Na-deoxycholate, 1% Triton X-100, 0.1% SDS, 1 mM DTT, 1 mM phenylmethylsulfonyl fluoride(PMSF), 1 µg/ml aprotinin and leupeptin, 1 mM NaF, 0.4 mM Na-orthovanadate, and 10 mM N-ethylmaleimide) to make a cell lysate. To the lysate containing a total protein content of 200 µg, was added an anti-Myc antibody, which had been used in the Western Blotting procedures, and the reaction was carried out for 2 h. After that, ProteinA beads (Amersham Pharmacia Biotech Corp.) were added to immunoprecipitate the anti-Myc antibody-bound Myc-Tcf-4 protein. Then, the recovered protein was detected by Western Blotting using an anti-Myc antibody and an anti-HA antibody (FIG.1B).

As a result, in the presence of Tcf-4, SUMO-1, and PIASy in HEK 293 cells, a shift in mobility due to conjugation of SUMO-1 to Tcf-4 was detected (FIG. 1A). It is believed that the shift in mobility results from the sumoylation of Tcf-4, because the change was not observed for inactive-form PIASyCA without SUMO activity, and conjugation of SUMO-1 with HA-tag was confirmed in the shifted band (FIG. 1B). Accordingly, it was revealed that sumoylation of Tcf-4 is accelerated by PIASy.

### Example 2

### (Detection of PIASy effects on transcriptional activity of Tcf-4)

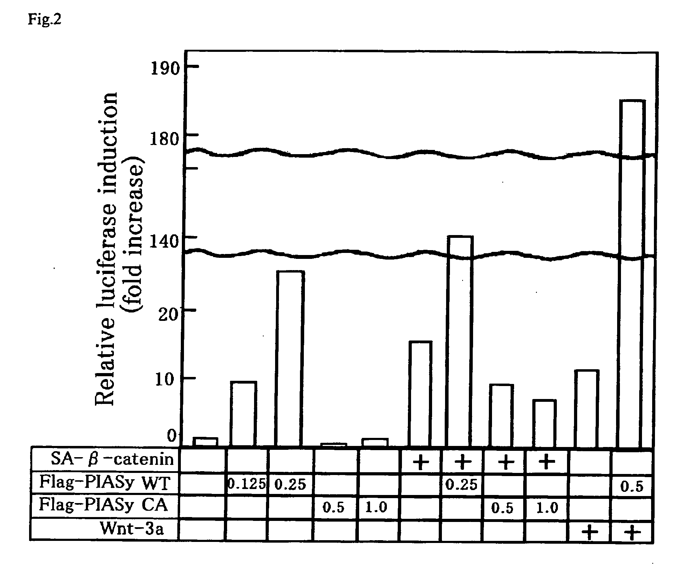

HEK 293 cells were seeded in the amount of 3 × 10⁵ cells in a 35 mm-diameter dish and incubated for 24 h. After incubation, the cells were transfected with a Tcf-responsive type plasmid (0.5 µg) inserted using LipofectAMINE 2000. At this time, a Wnt-3a expression plasmid (0.5 µg), a PIASy or PIASyCA expression plasmid, and an HA-β-catenineSA expression plasmid, were simultaneously added to some of the cells, according to the combinations and quantities (µg) shown in FIG 2. After the transfection with plasmids, incubation was performed for 40 h, and then the activity of firefly luciferase derived from the Tcf-responsive type plasmid, which was generated in the cells, was detected by a lumiphotometer manufactured by Futaba Medical Corp. (Tokyo), wherein PicaGene manufactured by Toyo Beanet Co. was used as a substrate. An error ascribable to a difference in transfection effectiveness was corrected by measuring the activity of pME18S/lacZ-derived β-galactosidase (FIG. 2).

In the HEK 293 cells, the reporter activity was enhanced by expression of β-catenin or Wnt-3. PIASy solely enhanced the reporter activity, and also synergistically increased the β-catenin- or Wnt-3-dependent reporter activity. On the other hand, inactive-form PIASyCA without sumoylation activity suppressed an increase of β-catenin-dependent reporter activity. Thus, it was revealed that PIASy solely enhances the transcriptional activity of Tcf-4 and also that PIASy synergistically increases the β-catenin-or Wnt-3-dependent transcriptional activity of Tcf-4.

### Example 3

### (Effects of PIASy, SUMO-1, or Axam on β-catenin-dependent transcriptional activity of Tcf-4)

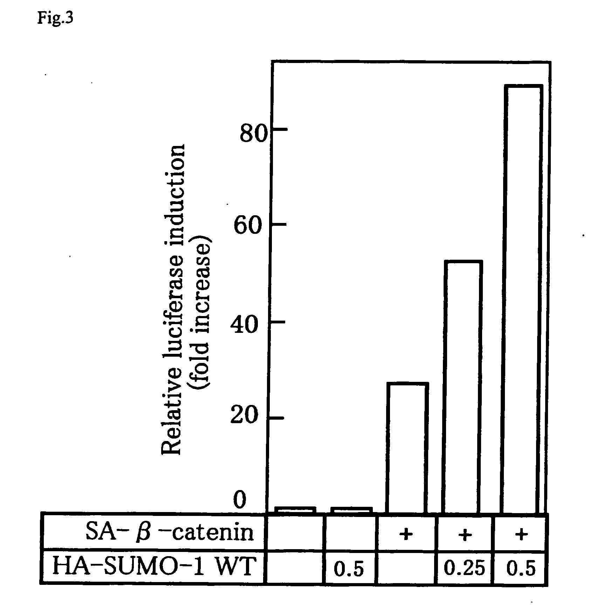

HEK293 cells were seeded in the amount of 3 × 10⁵ cells in a 35 mm-diameter dish and incubated for 24 h. After incubation, the cells were transfected with a Tcf-responsive type plasmid (0.5 µg), a pME18S/lacZ (0.5 µg), a Myc-Tcf-4 expression plasmid (0.1µg), and an HA-β-cateninSA expression plasmid (30 ng) using LipofectAMINE 2000. At that time, PIASy, SUMO-1, and Axam protein (in some cases, a mutant thereof) with desumoylation activity were simultaneously added in the amount of 0.5 µg. After incubation for 40 h, the activity of firefly luciferase derived from the Tcf-responsive type plasmid, which was generated in the cells, was detected by a lumiphotometer manufactured by Futaba Medical Corp. (Tokyo), wherein PicaGene manufactured by Toyo Beanet Co. was used as a substrate. Also, an error ascribable to the difference in transfection effectiveness was corrected by measuring the activity of pME18S/lacZ-derived β-galactosidase (FIGs. 3 to 5).

SUMO-1 synergistically accelerated the β-catenin-dependent transcriptional activity of Tcf-4 (FIG 3). Accordingly, it is considered that sumoylation is involved in the enhancement of transcriptional activity of Tcf-4.

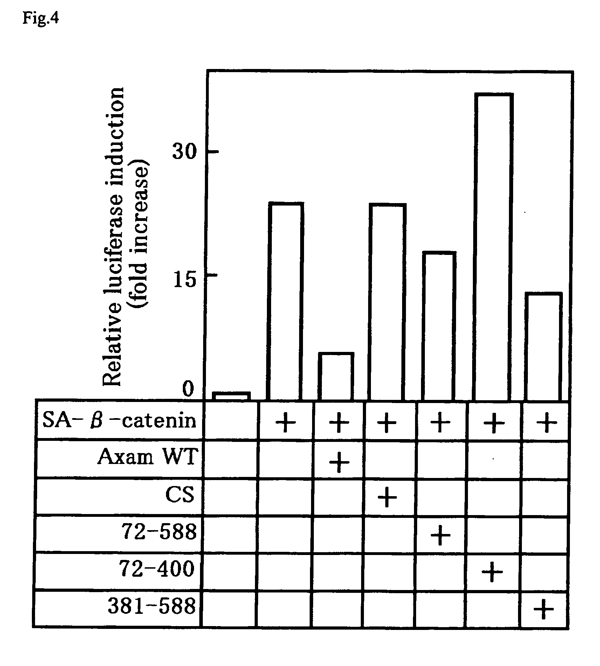

A full-length Axam protein with desumoylation activity showed suppression of the β-catenin-dependent transcriptional activity of Tcf-4 (FIG. 4 and FIG 5). In contrast, a mutant in which cysteine (C) at position 547 in the amino acid sequence of Axam had been substituted with serine (S) (a mutant exhibiting no desumoylation activity), did not suppress the β-catenin-dependent transcriptional activity of Tcf-4. Among the mutants of Axam in which amino acid residues were deleted at the N-terminal side, Axam (72-400) increased the β-catenin-dependent transcriptional activity of Tcf-4, while Axam (72-588) and Axam (381-588) showed suppression of the activity although their levels were weaker than that of the full-length Axam.

From the above results, it was revealed that the acceleration of sumoylation induces enhancement of the transcriptional activity of Tcf-4, and in contrast, the acceleration of desumoylation induces suppression of the transcriptional activity of Tcf-4. Therefore, the transcriptional activity of Tcf-4 can be suppressed by inhibiting Tcf-4 sumoylation, which enables the prevention and/or treatment of colon cancer, since it is reported that Tcf-4 is activated in colon cancer.

### Example 4

### (Detection of SUMO-1 conjugation to Tcf-4 in vitro)

cDNA coding for human Tcf-4 with GST-tag was inserted into a Baculovirus vector to construct a vector, and then GST-Tcf-4 was produced using Sf9 cells. GST-Aosl/His6-Uba2 (E1) was similarly produced using Sf-9 cells. SUMO-1GG (G denotes glycine), His6-Ubc9 (E2), MBP-PIASy (WT), and PIASy (PIASyCA: C342, 347A)(E3) were produced by Escherichia coli, after each cDNA had been inserted into a protein expression vector, respectively.

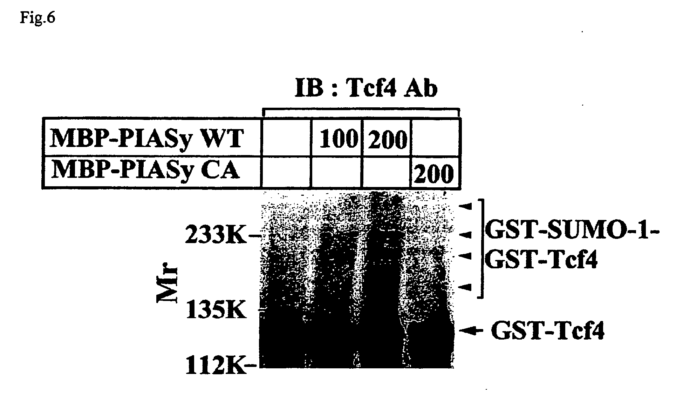

0.3 µg of GST-Tcf-4 , 0.5 µg of GST-Aos1/His6-Uba2 (E1), 0.5 µg of His6-Ubc9 (E2), and 10 µg of GST-SUMO-1GG were mixed in a sumoylation buffer solution (50 mM of Tris-HCl buffer, pH 7.5, 1 mM of MgCl₂, 2 mM of DTT, 5 mM of adenosine triphosphate (ATP)) to react at 30°C for 3 h. After that, the Tcf-4 protein was detected by Western Blotting using an anti-Tcf-4 antibody. However, a shift in mobility ascribable to the conjugation of SUMO-1 to Tcf-4 was not detected. When 0.1 µg or 0.2 µg of MBP-PIASy was further added thereto and the Tcf-4 protein was detected similarly, a shift in the mobility was observed. However, a shift in mobility was not detected when MBP-PIASyCA that does not have binding ability to Ubc9 (FIG. 6) was added. These findings proved that Tcf-4 is sumoylated by PIASy in vitro.

### Example 5

### (Detection of SUMO-1 conjugation to Tcf-4 by PIAS)

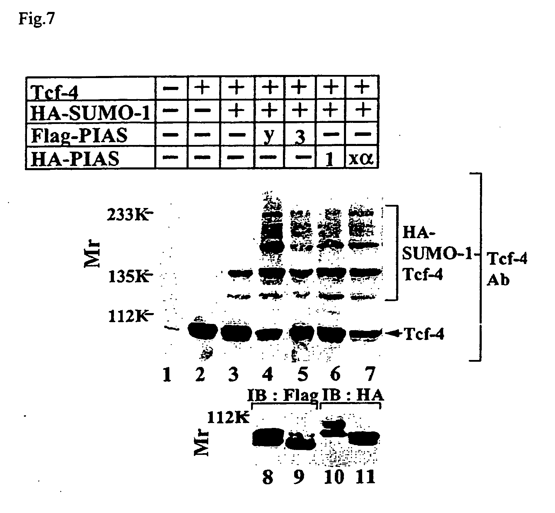

A human Tcf-4 expression plasmid, a Flag-PIAS3 expression plasmid, an HA-PIAS1 expression plasmid, and an HA-PIASxα expression plasmid were constructed using a recombinant DNA technique. Similarly to Example 1, these plasmids, together with an HA-SUMO-1 expression plasmid, were transfected in HEK 293 cells. After incubation, the cells were treated with 10% TCA to give a precipitate. The precipitate was subjected to electrophoresis to detect the sumoylation of Tcf-4 using an anti-Tcf-4 antibody, anti-Flag antibody, and anti-HA antibody (FIG. 7).

As a result, in the presence of Tcf-4 and SUMO-1 in the HEK 293 cells, a shift in mobility due to PIASy, PIAS1, PIAS3, and PIASxα was detected (FIG. 7, lanes 4 to 7). This shift in mobility results from the conjugation of SUMO-1 to Tcf-4. It has thus been proved that the sumoylation of Tcf-4 is accelerated by not only PIASy, but also by PIAS1, PIAS3, and PIASxα.

### INDUSTRIAL APPLICABILITY

In the present invention, it has been found that transcriptional factor Tcf-4 is sumoylated by PIAS, such as PIASy, PIAS1, PIAS3, or PIASxα, and that the sumoylation enhances the Tcf-4 function. It is known that Tcf-4 is a transcriptional factor that is located downstream of a β-catenin signal, and that it is activated in colon cancer. Thus, inhibiting the sumoylation of Tcf-4 by PIAS or desumoylating sumoylated Tcf-4 can suppress the enhanced transcriptional activity of Tcf-4, which may allow the prevention and/or treatment of colon cancer. The present invention is therefore extremely useful for preventing and/or treating colon cancer, and for research concerning sumoylation or a β-catenin/Tcf signal transduction system.

## Claims

1. A method for identifying a compound that inhibits sumoylation of Tcf-4 by PIASy, (protein inhibitor of activated STAT), comprising the steps of:
(i) contacting PIASy and/or Tcf-4 with a test compound under conditions allowing for the interaction of the test compound with PIASy and/or Tcf-4; and
(ii) determining whether the test compound inhibits the sumoylation of Tcf-4 by PIASy **in a system where PIASy, Tef-4 and small ubiquitin-like modifier (SUMO) are employed to induce sumoylation,** by employing a system that uses a signal and/or marker capable of detecting sumoylation of Tcf-4 to detect the presence, absence, or alteration of the signal and/or marker.

2. A method for identifying a compound that inhibits sumoylation of Tcf-4 by PIASy, comprising the steps of: (i) contacting the cells described in (A), (B), (C), or (D) below with a test compound; and (ii) determining whether the test compound inhibits the sumoylation of Tcf-4 by PIASy, by detecting sumoylated Tcf-4 or by detecting the presence, absence or alteration of a signal and/or marker for detecting sumoylation of Tcf-4; wherein cells (A), (B), (C), and (D) are as follows:
(A) cells that have co-expressed PIASy, small ubiquitin-like modifier (SUMO), and Tcf-4 to which a signal and/or marker for detecting sumoylation of Tcf-4 is introduced;
(B) cells that have coexpressed PIASy, Tcf-4, and SUMO to which a signal and/or marker for detecting sumoylation of Tcf-4 is introduced;
(C) cells that have coexpressed PIASy, Tcf-4 to which a signal and/or marker for detecting sumoylation of Tcf-4 is introduced , and SUMO to which the signal and/or marker is introduced; and
(D) cells that have co-expressed PIASy, SUMO, and Tcf-4.

3. A method for identifying a compound that inhibits sumoylation of Tcf-4 by PIASy, comprising the steps of: (i) contacting the cells described in (E) or (F) below with a test compound; and (ii) determining whether the test compound inhibits the sumoylation of Tcf-4 by PIASy, by measuring a fluorescence intensity of a green fluorescent protein (GFP) after adding a luciferase substrate; wherein cells (E) and (F) are as follows:
(E) cells that have coexpressed a fused protein of Tcf-4 and GFP, a fused protein of small ubiquitin-like modifier (SUMO) and luciferase, and PIASy; and
(F) cells that have co-expressed a fused protein of Tcf-4 and luciferase, a fused protein of SUMO and GFP, and PIASy.

4. A method for identifying a compound that inhibits sumoylation of Tcf-4 by PIASy, comprising the steps of: (i) bringing PIASy, E1 SUMO-activating enzyme, E2 SUMO-conjugation enzyme, small ubiquitin-like modifier (SUMO) or SUMO to which a signal and/or marker for detecting sumoylation of Tcf-4 is introduced, and a test compound, to contact with Tcf-4 or Tcf-4 immobilised on a solid phase; and (ii) determining whether the test compound inhibits the sumoylation of Tcf-4 by PIASy, by detecting sumoylated Tcf-4 or by detecting the presence, absence, or alteration of the signal and/or marker.

5. A method for identifying a compound that inhibits sumoylation of Tcf-4 by PIASy, comprising the steps of: (i) bringing PIASy, E1 SUMO-activating enzyme, E2 SUMO-conjugation enzyme, small ubiquitin-like modifier labelled with a radioactive isotope, biotin or a peptide-tag, and a test compound to contact with Tcf-4 immobilised to a solid phase; and (ii) determining whether the test compound inhibits the sumoylation of Tcf-4 by PIASy by measuring the substance used for labelling.

6. A method for identifying a compound that inhibits sumoylation of Tcf-4 by PIASy, comprising the steps of: (i) bringing PIASy, E1 SUMO-activating enzyme, E2 SUMO-conjugation enzyme, small ubiquitin-like modifier labelled with europium, Tcf-4 labelled with a substance that radiates secondary fluorescence upon excitation by fluorescence irradiated by europium, and a test compound to reaction; and (ii) determining whether the test compound inhibits the sumoylation of Tcf-4 by PIASy, by irradiating light of a wavelength that excites europium and then measuring the secondary fluorescence intensity.

7. A method for identifying a compound that inhibits sumoylation of Tcf-4 by PIASy, comprising the steps of: (i) bringing PIASy, E1 SUMO-activating enzyme, E2 SUMO-conjugation enzyme, and a test compound to react with a fused protein of Tcf-4 and luciferase as well as a fused protein of small ubiquitin-like modifier (SUMO) and a green fluorescent protein (GFP), or with a fused protein of Tcf-4 and GFP as well as a fused protein of SUMO and luciferase; and (ii) determining whether the test compound inhibits the sumoylation of Tcf-4 by PIASy, by measuring the fluorescence intensity of the GFP after adding a luciferase substrate.

## Patentansprüche

1. Verfahren zur Identifizierung einer Verbindung, welche die Sumoylierung von Tcf-4 durch PIASy (Proteininhibitor von aktiviertem STAT) inhibiert, umfassend die Schritte von:
(i) in Kontakt bringen von PIASy und/oder Tcf-4 mit einer Testverbindung unter Bedingungen, die die Interaktion der Testverbindung mit PIASy und/oder Tcf-4 erlauben; und
(ii) Bestimmen, ob die Testverbindung die Sumoylierung von Tcf-4 durch PIASy in einem System inhibiert, in dem PIASy, Tcf-4 und kleiner Ubiquitin-ähnlicher Modifikator (SUMO) verwendet werden, um die Sumoylierung zu induzieren, durch Verwenden eines Systems, das ein Signal und/oder Marker verwendet, der in der Lage ist, die Sumoylierung von Tcf-4 nachzuweisen, um die Anwesenheit, Abwesenheit, oder Veränderung des Signals und/oder Markers nachzuweisen.

2. Verfahren zur Identifizierung einer Verbindung, welche die Sumoylierung von Tcf-4 durch PIASy inhibiert, umfassend die Schritte von: (i) in Kontakt bringen der in (A), (B), (C) oder (D) unten beschriebenen Zellen mit einer Testverbindung; und (ii) Bestimmen, ob die Testverbindung die Sumoylierung von Tcf-4 durch PIASy inhibiert, durch Nachweisen von sumoyliertem Tcf-4 oder durch Nachweisen der Anwesenheit, Abwesenheit, oder Veränderung eines Signals und/oder Markers zum Nachweis der Sumoylierung von Tcf-4; wobei die Zellen (A), (B), (C) und (D) wie folgt sind:
(A) Zellen, die PIASy, kleiner Ubiquitin-ähnlicher Modifikator (SUMO) und Tcf-4 koexprimieren, an das ein Signal und/oder Marker zum Nachweis der Sumoylierung von Tcf-4 eingefügt ist;
(B) Zellen, die PIASy, Tcf-4 und SUMO koexprimieren, an den ein Signal und/oder Marker zum Nachweis der Sumoylierung von Tcf-4 eingefügt ist;
(C) Zellen, die PIASy, Tcf-4, an das ein Signal und/oder Marker zum Nachweis der Sumoylierung von Tcf-4 eingefügt ist, und SUMO koexprimieren, an den ein Signal und/oder Marker eingefügt ist; und
(D) Zellen, die PIASy, SUMO und Tcf-4 koexprimieren.

3. Verfahren zur Identifizierung einer Verbindung, welche die Sumoylierung von Tcf-4 durch PIASy inhibiert, umfassend die Schritte von: (i) in Kontakt bringen der in (E) oder (F) unten beschriebenen Zellen mit einer Testverbindung und (ii) Bestimmen, ob die Testverbindung die Sumoylierung von Tcf-4 durch PIASy inhibiert, durch Messen einer Fluoreszenz-Intensität eines grün fluoreszierenden Proteins (GFP) nach der Hinzugabe eines Luziferase-Substrats; wobei die Zellen (E) und (F) wie folgt sind:
(E) Zellen, die ein fusioniertes Protein von Tcf-4 und GFP, ein fusioniertes Protein von kleiner Ubiquitin-ähnlicher Modifikator (SUMO) und Luziferase und PIASy koexprimieren; und
(F) Zellen, die ein fusioniertes Protein von Tcf-4 und Luziferase, ein fusioniertes Protein von SUMO und GFP und PIASy koexprimieren.

4. Verfahren zur Identifizierung einer Verbindung, welche die Sumoylierung von Tcf-4 durch PIASy inhibiert, umfassend die Schritte von: (i) in Kontakt bringen von PIASy, E1 SUMO-aktivierendem Enzym, E2 SUMO-Konjugationsenzym, kleiner Ubiquitin-ähnlicher Modifikator (SUMO) oder SUMO, an den ein Signal und/oder Marker zum Nachweis der Sumoylierung von Tcf-4 eingefügt ist, und eine Testverbindung, um mit Tcf-4 oder Tcf-4 immobilisiert auf einer festen Phase in Kontakt gebracht zu werden; und (ii) Bestimmen, ob die Testverbindung die Sumoylierung von Tcf-4 durch PIASy inhibiert, durch Nachweisen von sumoyliertem Tcf-4 oder durch Nachweisen der Anwesenheit, Abwesenheit, oder Veränderung des Signals und/oder Markers.

5. Verfahren zur Identifizierung einer Verbindung, welche die Sumoylierung von Tcf-4 durch PIASy inhibiert, umfassend die Schritte von: (i) in Kontakt bringen von PIASy, E1 SUMO-aktivierendem Enzym, E2 SUMO-Konjugationsenzym, kleiner Ubiquitin-ähnlicher Modifikator markiert mit einem radioaktiven Isotop, Biotin oder einem Peptidtag und einer Testverbindung um mit Tcf-4 immobilisiert auf einer festen Phase in Kontakt gebracht zu werden; and (ii) Bestimmen, ob die Testverbindung die Sumoylierung von Tcf-4 durch PIASy inhibiert, durch Messen der zur Markierung verwendeten Substanz.

6. Verfahren zur Identifizierung einer Verbindung, welche die Sumoylierung von Tcf-4 durch PIASy inhibiert, umfassend die Schritte von: (i) zur Reaktion bringen von PIASy, E1 SUMO-aktivierendem Enzym, E2 SUMO-Konjugationsenzym, kleiner Ubiquitin-ähnlicher Modifikator markiert mit Europium, Tcf-4 markiert mit einer Substanz, die sekundäre Fluoreszenz nach Anregung durch Fluoreszenz bestrahlt durch Europium abstrahlt, und einer Testverbindung; und (ii) Bestimmen, ob die Testverbindung die Sumoylierung von Tcf-4 durch PIASy inhibiert, durch Bestrahlen mit Licht einer Wellenlänge, die Europium anregt, und dann Messen der sekundären Fluoreszenzintensität.

7. Verfahren zur Identifizierung einer Verbindung, welche die Sumoylierung von Tcf-4 durch PIASy inhibiert, umfassend die Schritte von: (i) zur Reaktion bringen von PIASy, E1 SUMO-aktivierendem Enzym, E2 SUMO-Konjugationsenzym und einer Testverbindung mit einem fusionierten Protein von Tcf-4 und Luziferase sowie einem fusionierten Protein von kleiner Ubiquitin-ähnlicher Modifikator (SUMO) und einem grün fluoreszierenden Protein (GFP), oder mit einem fusionierten Protein von Tcf-4 and GFP sowie einem fusionierten Protein von SUMO und Luziferase; und (ii) Bestimmen, ob die Testverbindung die Sumoylierung von Tcf-4 durch PIASy inhibiert, durch Messen der Fluoreszenzintensität des GFPs nach Hinzugabe eines Luziferase-Substrats.

## Revendications

1. Procédé pour identifier un composé qui inhibe la sumoylation de Tcf-4 par PIASy (Protein Inhibitor of Activated STAT - Inhibiteur de protéines de STAT activés), comprenant les étapes suivantes :
(i) mettre en contact PIASy et/ou Tcf-4 avec un composé test dans des conditions permettant l'interaction du composé test avec PIASy et/ou Tcf-4 ; et
(ii) déterminer si le composé test inhibe la sumoylation de Tcf-4 par PIASy **dans un système où PIASy, Tcf-4 et un modificateur de type ubiquitine (SUMO - Small Ubiquitin-like Modifier) sont utilisés** pour induire la sumoylation, au moyen d'un système qui utilise un signal et/ou marqueur capable de détecter la sumoylation de Tcf-4, pour détecter la présence, l'absence ou l'altération du signal et/ou du marqueur.

2. Procédé pour identifier un composé qui inhibe la sumoylation de Tcf-4 par PIASy, comprenant les étapes suivantes : (i) mettre en contact les cellules décrites en (A), (B), (C), ou (D) ci-dessous avec un composé test ; et (ii) déterminer si le composé test inhibe la sumoylation de Tcf-4 par PIASy, en détectant le Tcf-4 sumoylé ou en détectant la présence, l'absence ou l'altération d'un signal et/ou marqueur de détection de la sumoylation de Tcf-4 ; les cellules (A), (B), (C), et (D) se présentant comme suit :
(A) cellules ayant co-exprimé PIASy, le modificateur de type ubiquitine (SUMO), et Tcf-4 dans lequel est introduit un signal et/ou marqueur pour détection de la sumoylation de Tcf-4 ;
(B) cellules ayant co-exprimé PIASy, Tcf-4, et SUMO dans lequel est introduit un signal et/ou marqueur pour détection de la sumoylation de Tcf-4 ;
(C) cellules ayant co-exprimé PIASy, Tcf-4 dans lequel est introduit un signal et/ou marqueur pour détection de la sumoylation de Tcf-4, et SUMO dans lequel est introduit le signal et/ou marqueur ; et
(D) cellules ayant co-exprimé PIASy, SUMO et Tcf-4.

3. Procédé pour identifier un composé qui inhibe la sumoylation de Tcf-4 par PIASy, comprenant les étapes suivantes : (i) mettre en contact les cellules décrites en (E) ou (F) ci-dessous avec un composé test ; et (ii) déterminer si le composé test inhibe la sumoylation de Tcf-4 par PIASy, en mesurant une intensité de fluorescence d'une protéine verte fluorescente (GFP) après addition d'un substrat de luciférase ; les cellules (E) et (F) se présentant comme suit :
(E) cellules ayant co-exprimé une protéine fusionnée de Tcf-4 et GFP, une protéine fusionnée de SUMO et de luciférase, et PIASy ; et
(F) cellules ayant co-exprimé une protéine fusionnée de Tcf-4 et luciférase, une protéine fusionnée de SUMO et GFP, et PIASy.

4. Procédé pour identifier un composé qui inhibe la sumoylation de Tcf-4 par PIASy, comprenant les étapes suivantes : (i) mettre PIASy, l'enzyme d'activation SUMO E1, l'enzyme de conjugaison SUMO E2, le modificateur de type ubiquitine (SUMO) ou SUMO dans lequel est introduit un signal et/ou un marqueur pour détection de sumoylation de Tcf-4, et un composé test, en contact avec Tcf-4 ou Tcf-4 immobilisé sur une phase solide ; et (ii) déterminer si le composé test inhibe la sumoylation de Tcf-4 par PIASy, en détectant le Tcf-4 sumoylé ou en détectant la présence, l'absence ou l'altération du signal et/ou marqueur.

5. Procédé pour identifier un composé qui inhibe la sumoylation de Tcf-4 par PIASy, comprenant les étapes suivantes : (i) mettre PIASy, l'enzyme d'activation SUMO E1, l'enzyme de conjugaison SUMO E2, le modificateur de type ubiquitine marqué à l'aide d'un isotope radioactif, de biotine ou d'un marqueur de peptide, et un composé test, en contact avec Tcf-4 immobilisé sur une phase solide ; et (ii) déterminer si le composé test inhibe la sumoylation de Tcf-4 par PIASy en mesurant la substance utilisée pour le marquage.

6. Procédé pour identifier un composé qui inhibe la sumoylation de Tcf-4 par PIASy, comprenant les étapes suivantes : (i) mettre en réaction PIASy, l'enzyme d'activation SUMO E1, l'enzyme de conjugaison SUMO E2, le modificateur de type ubiquitine marqué à l'europium, Tcf-4 marqué à l'aide d'une substance qui émet une fluorescence secondaire lors de l'excitation par fluorescence émise par l'europium, et un composé test ; et (ii) déterminer si le composé test inhibe la sumoylation de Tcf-4 par PIASy, en irradiant au moyen de lumière d'une longueur d'onde qui excite l'europium, puis en mesurant l'intensité de fluorescence secondaire.

7. Procédé pour identifier un composé qui inhibe la sumoylation de Tcf-4 par PIASy, comprenant les étapes suivantes : (i) mettre en réaction PIASy, l'enzyme d'activation SUMO E1, l'enzyme de conjugaison SUMO E2, et un composé test avec une protéine fusionnée de Tcf-4 et luciférase ainsi qu'une protéine fusionnée de modificateur de type ubiquitine (SUMO) et d'une protéine verte fluorescente (GFP), ou avec une protéine fusionnée de Tcf-4 et GFP ainsi qu'une protéine fusionnée de SUMO et luciférase; et (ii) déterminer si le composé test inhibe la sumoylation de Tcf-4 par PIASy, en mesurant l'intensité de fluorescence de la GFP après addition d'un substrat de luciférase.
